# EUROPÄISCHE PATENTSCHRIFT

(11) **EP 2 282 302 B1**
(45) Veröffentlichungstag und Bekanntmachung des Hinweises auf die Patenterteilung: **19.11.2014**
(21) Anmeldenummer: 10169559.1
(22) Anmeldetag: 14.07.2010
(51) Int. Cl.: G09F 3/02, A61M 5/31, G09F 3/10

(54) **Etikett, Verwendung eines erhabenen Strukturmerkmals, Injektionsvorrichtung und Verfahren zur Herstellung eines Etiketts**
Label, use of an embossed structure element, injection device and method for producing a label
Etiquette, utilisation d'une caractéristique structurelle élevée, dispositif d'injection et procédé de fabrication d'une étiquette

(30) Priorität: 27.07.2009 DE 102009034823
(43) Veröffentlichungstag der Anmeldung: 09.02.2011
(73) Patentinhaber: Schreiner Group GmbH & Co. KG, 85764 Oberschleissheim (DE)
(72) Erfinder: Licha, Robert, 85521 Ottobrunn (DE)
(74) Vertreter: Epping - Hermann - Fischer

(56) Entgegenhaltungen:
- EP-A1- 1 566 786
- EP-A2- 1 477 196
- WO-A1-98/19289
- DE-A1-102006 048 209

## Beschreibung

Die Erfindung betrifft ein Etikett, die Verwendung eines erhabenen Strukturmerkmals, eine Injektionsvorrichtung und ein Verfahren zur Herstellung eines Etiketts.

Etiketten werden oftmals zur Etikettierung von medizinischen Behältnissen, beispielsweise bei Medikamentenampullen für Injektionspens eingesetzt. Dabei dienen bisher bekannte Etiketten lediglich der Kennzeichnung des in dem medizinischen Behältnis enthaltenen Wirkstoffs. Das medizinische Behältnis wird in dem Injektionspen befestigt. Üblicherweise weisen dabei die Medikamentenampullen glatte Seitenwände aus Glas auf, die nur wenig oder keinerlei Befestigungsmöglichkeiten bieten, so dass es mitunter schwierig ist, medizinische Behältnisse in einem medizinischen Gerät, wie beispielsweise einem Injektionspen zu befestigen.

Die Druckschrift EP 1566786 A1 betrifft ein Etikett zum Übersiegeln eines Übergangs zwischen axial verschiedenen Querschnitten von Körpern, wobei der Querschnittsunterschied durch auf dem Etikett angeordnete Distanzstücke ausgeglichen wird.

Es besteht daher in der Technik ein Bedarf, weitere Befestigungsmöglichkeiten zu schaffen.

Diese Aufgabe wird in einem ersten Aspekt durch ein Etikett zum Etikettieren eines Behältnisses gelöst, das eine flexible Trägerschicht umfasst, die auf einer Seite wenigstens teilweise mit wenigstens einem erhabenen Strukturelement versehen ist, wobei das Strukturelement derart ausgeführt ist, dass im Falle des Aufbringens des Etiketts auf das Behältnis das Strukturelement vom Behältnis weggerichtet ist und wobei das Strukturelement dazu geeignet ist, mit einem weiteren Strukturelement eine formschlüssige Verbindung zu bilden.

Durch das Etikett wird die Möglichkeit geschaffen, im Falle des Aufbringens des Etiketts auf ein Behältnis, ein Halteelement für das Behältnis mit dem Etikett zu bilden, sodass auch Behältnisse, die keinerlei weitere Haltemöglichkeiten aufweisen, beispielsweise in einem Gehäuse oder irgendeiner anderen Befestigung, angebracht werden können. Das erhabene Strukturelement kann dabei sowohl die alleinige als auch eine zusätzliche Haltemöglichkeit darstellen. Insgesamt ergibt sich ein Etikett mit der Zusatzfunktion des Bildens eines Halteelements, sodass auf kostengünstige und einfache Weise mehrere Funktionen in einem Etikett integriert sind, ohne jedoch den Herstellungsprozess des Etiketts signifikant verkomplizieren zu müssen.

Gemäß einer weiteren Ausführungsform des Etiketts weist das Strukturelement eine Höhe auf, die größer als das Vierfache der Trägerschicht ist und vorzugsweise größer als das Zwanzigfache der Trägerschicht ist. Das Strukturelement kann beispielsweise eine Höhe aufweisen, die größer als 0,4 mm ist und vorzugsweise zwischen 0,4 mm und 2,0 mm beträgt. Wenn das Etikett mit dem Strukturelement auf ein Behältnis, beispielsweise auf eine Medikamentenampulle, aufgebracht wird und das Behältnis in ein weiteres Behältnis, beispielsweise den Hohlkörper einer Spritze, das ebenfalls weitere Strukturelemente aufweist, eingeschoben wird, ist sichergestellt, dass das Strukturelement des Etiketts mit dem weiteren Strukturelement einen stabilen Anschlag und somit eine formschlüssige Verbindung bildet. Das Strukturelement kann mit einer Seitenfläche, die durch Seiten des Strukturelements begrenzt wird, auf der Trägerschicht aufgebracht sein. Das Strukturelement kann bei dieser Ausführungsform eine Höhe aufweisen, die mindestens doppelt so groß ist, wie die kürzere der Seiten der Seitenfläche.

In einer weiteren Ausführungsform ist eine Vielzahl von Strukturelementen auf der Seite der Trägerschicht angebracht, wobei im Falle des Aufbringens des Etiketts auf das Behältnis eine Vielzahl von vom Behältnis weggerichteten Halteelementen entsteht. Die Halteelemente können beispielsweise als Vorsprünge ausgebildet sein. Dadurch kann die Haltefähigkeit des Etiketts zusätzlich gesteigert werden, was insbesondere dann erstrebenswert ist, wenn im Falle des Aufbringens des Etiketts auf das Behältnis dieses größeren Kräften ausgesetzt ist.

Gemäß einer weiteren Ausführungsform weist jedes der Strukturelemente der Vielzahl der Strukturelemente eine Seitenfläche auf. Die Strukturelemente sind derart auf der Trägerschicht angeordnet, dass die jeweilige Seitenfläche der Strukturelemente in einer Ebene angeordnet ist. Wenn das Etikett mit den Strukturelementen auf einem Körper, beispielsweise einem zylinderförmigen Behältnis, aufgebracht ist, bilden die jeweiligen Seitenflächen der Strukturelemente eine Anschlagfläche, die in einer Ebene liegt. Ein weiterer Körper, der ebenfalls Strukturelemente aufweist, lässt sich derart anordnen, dass die weiteren Strukturelemente in der Ebene, in der die Seitenflächen enthalten sind, an den Strukturelementen des Etiketts anliegen. Somit wird zwischen dem Körper, auf dem das Etikett angebracht ist, und dem Körper, an dem die weiteren Strukturelemente angeordnet sind, eine formschlüssige Verbindung gebildet.

Das Strukturelement kann beispielsweise mittels einer Klebeschicht auf die Trägerschicht geklebt sein. Bei der Klebeschicht kann es sich um eine Schicht handeln, die bei Einwirkung von Wärme oder Strahlung aushärtet.

In einer weiteren Ausführungsform ist das Strukturelement als Kunststoffsteg auf die Trägerschicht aufgebracht. Kunststoffstege stellen eine kostengünstige und einfache Möglichkeit dar, erhabene Strukturelemente zu bilden, wobei gemäß dieser Vorgehensweise auch eine Integration in bestehende Fabrikationsanlagen für die Etikettenherstellung möglich ist.

In einer weiteren Ausführungsform ist der Kunststoffsteg mit der Trägerschicht verklebt. Durch die Verklebung wird eine kostengünstige und einfache Verbindung zwischen Kunststoffsteg und Trägerschicht geschaffen, die ebenfalls in bestehende Fabrikationsanlagen für die Etikettenherstellung integriert werden kann.

Gemäß einer weiteren Ausführungsform ist das Strukturelement als strukturierte Farbschicht auf die Trägerschicht aufgebracht. Die strukturierte Farbschicht kann beispielsweise auf die Trägerschicht mittels eines Lacks bedruckt sein. Bedruckung stellt einen Standardschritt bei der Etikettenherstellung dar, sodass die Prozesskomplexität gering bleibt und somit eine kostengünstige und einfache Möglichkeit der Etikettenherstellung geschaffen wird.

In einer weiteren Ausführungsform entspricht der Lack einer Druckfarbe für Blindenschrift. Druckfarben für Blindenschrift bilden nach Bedruckung und Aushärtung Strukturelemente, die sich von ihrem Untergrund deutlich abheben, sodass eine kostengünstige und einfache Möglichkeit geschaffen wird, erhabene Strukturelemente zu bilden.

Gemäß einer weiteren Ausführungsform ist das Strukturelement als strukturierte Folienschicht auf die Trägerschicht aufgebracht. Das Aufbringen von Folienschichten stellt ebenfalls einen Standardschritt bei der Etikettenherstellung dar, sodass die Prozesskomplexität gering bleibt und somit eine kostengünstige und einfache Möglichkeit der Etikettenherstellung geschaffen wird.

In einer weiteren Ausführungsform ist die strukturierte Folienschicht auf die Trägerschicht aufgeklebt. Durch die Verklebung wird eine kostengünstige und einfache Verbindung zwischen Folienschicht und Trägerschicht geschaffen, die ebenfalls in bestehende Fabrikationsanlagen für die Etikettenherstellung integriert werden kann.

In einer weiteren Ausführungsform umfasst die flexible Trägerschicht darüber hinaus Symbole, die eine Information über den Inhalt des Behältnisses, Bedienungshinweise oder Herstellerhinweise repräsentieren. In einer weiteren Ausführungsform sind die Symbole auf die flexible Trägerschicht gedruckt. Bedruckung stellt einen Standardschritt bei der Etikettenherstellung dar, sodass die Prozesskomplexität gering bleibt und somit eine kostengünstige und einfache Möglichkeit der Etikettenherstellung geschaffen wird.

In einer weiteren Ausführungsform ist das Etikett mittels einer Klebeschicht, die auf einer der Seite der Trägerschicht gegenüberliegenden weiteren Seite aufgebracht ist, an dem Behältnis befestigbar.

In einer weiteren Ausführungsform ist das erhabene Strukturelement im Falle des Aufbringens des Etiketts auf das Behältnis als Widerlager für die Befestigung des Behältnisses in einem Gehäuse geeignet.

In einer weiteren Ausführungsform sind mehrere Strukturelemente auf der Trägerschicht parallel zueinander aufgebracht. In einer weiteren Ausführungsform weisen die parallelen Strukturelemente jeweils einen Startpunkt auf und sind entlang einer Linie angeordnet.

Gemäß einer weiteren Ausführungsform ist das Behältnis zylinderförmig ausgebildet und das Strukturelement ist entlang einer axialen Richtung des Behältnisses angeordnet.

In einer weiteren Ausführungsform bildet das Strukturelement im Falle des Aufbringens des Etiketts auf das Behältnis ein Gewinde.

In einer weiteren Ausführungsform bilden im Falle des Aufbringens des Etiketts Zwischenräume des Strukturelements das Gewinde.

In einer weiteren Ausführungsform entspricht eine Außenabmessung des Etiketts einem Außenumfang des Behältnisses.

Gemäß einer weiteren Ausführungsform wird die Vielzahl der Strukturelemente aus einer Vielzahl von kugelförmigen Elementen gebildet, wobei die kugelförmigen Elemente derart auf der Seite der Trägerschicht angeordnet sind, dass die kugelförmigen Elemente im Falle des Aufbringens des Etiketts auf das Behältnis das Gewinde bilden.

Gemäß einem weiteren Aspekt lässt sich wenigstens ein erhabenes Strukturelement auf einem Etikett verwenden, wobei das Etikett auf einer Außenseite eines zylindrischen Behältnisses derart angeordnet ist, dass das Strukturelement von der Außenseite des Behältnisses weggerichtet ist und zur Bildung einer formschlüssigen Verbindung mit einem weiteren Strukturelement geeignet ist. Das Strukturelement kann auf dem Etikett in der Weise verwendet werden, dass das Strukturelement eine Haltefunktion in Form eines Widerlagers oder Gewindes für das zylindrische Behältnis in einem Gehäuse ausübt.

Die Aufgabe wird in einem weiteren Aspekt durch eine medizinische Injektionsvorrichtung gelöst, die Folgendes umfasst:
- ein Etikett, das eine flexible Trägerschicht aufweist, die auf einer Seite wenigstens teilweise mit wenigstens einem erhabenen Strukturelement versehen ist,
- ein zylindrisches Behältnis zur Aufnahme eines medizinischen Produktes, das mit dem Etikett versehen ist, wobei das Etikett mit einer der Seite gegenüberliegenden Seite an dem Behältnis angeordnet ist und das Strukturelement einen vom Behältnis weggerichteten Vorsprung bildet und
- ein Gehäuse, in dem das Behältnis angeordnet ist, wobei das Strukturelement dazu geeignet ist, mit einem weiteren Strukturelement eine formschlüssige Verbindung zu bilden.

Gemäß einer weiteren Ausführungsform übt das Strukturelement eine Haltefunktion in Form eines Widerlagers oder Gewindes für das zylindrische Behältnis in dem Gehäuse aus.

Gemäß einer weiteren Ausführungsform weist das Strukturelement eine Höhe auf, die größer als das Vierfache der Trägerschicht ist. Das Strukturelement kann beispielsweise eine Höhe aufweisen, die größer als 0,4 mm ist und vorzugsweise zwischen 0,4 mm und 2 mm beträgt. Das Strukturelement kann mit einer Seitenfläche, die durch Seiten des Strukturelements begrenzt wird, auf der Trägerschicht aufgebracht sein. Das Strukturelement weist bei dieser Ausführungsform eine Höhe auf, die mindestens doppelt so groß ist, wie die kürzere der Seiten der Seitenfläche.

Gemäß einer weiteren Ausführungsform ist eine Vielzahl von Strukturelementen auf der Seite der Trägerschicht angebracht, wobei im Falle des Aufbringens des Etiketts auf das Behältnis eine Vielzahl von vom Behältnis weggerichteten Halteelementen entsteht.

Gemäß einer weiteren Ausführungsform der medizinischen Injektionsvorrichtung ist das Strukturelement mittels einer Klebeschicht auf die Trägerschicht geklebt. Das zylindrische Behältnis kann beispielsweise eine Medikamentenampulle oder eine Medikamentenkartusche sein. Gemäß einer weiteren Ausführungsform kann das Gehäuse Teil eines Injektionspens oder einer Spritze sein. Das Behältnis kann beispielsweise an einem distalen Ende eine Nadel aufweisen, die von einer Nadelschutzvorrichtung umgeben ist.

Im Folgenden wird ein Verfahren zur Herstellung eines Etiketts angegeben, das die folgenden Schritte umfasst:
- Bereitstellen einer flexiblen Trägerschicht und Aufbringen wenigstens eines erhabenen Strukturelements auf einer Seite der Trägerschicht, wobei das erhabene Strukturelement derart ausgeführt ist, dass im Falle des Aufbringens auf das Behältnis das erhabene Strukturelement vom Behältnis weggerichtet ist und wobei das Strukturelement dazu geeignet ist, mit einem weiteren Strukturelement eine formschlüssige Verbindung zu bilden.

Gemäß dem Verfahren ist es weiterhin vorgesehen, dass das Strukturelement mit einer Höhe auf der Trägerschicht aufgebracht wird, die größer als das Vierfache der Trägerschicht ist. Das Strukturelement kann beispielsweise mit einer Höhe, die größer als 0,4 mm ist und vorzugsweise zwischen 0,4 mm und 2 mm beträgt, auf der Trägerschicht aufgebracht werden. Gemäß einer Weiterbildung des Verfahrens kann das Strukturelement mit einer Seitenfläche, die durch Seiten des Strukturelements begrenzt wird, auf der Trägerschicht aufgebracht werden. Das Strukturelement kann mit einer Höhe auf der Trägerschicht aufgebracht werden, die mindestens doppelt so groß ist, wie die kürzere der Seiten der Seitenfläche. Das Strukturelement kann beispielsweise mittels einer Klebeschicht auf die Trägerschicht aufgeklebt werden.

Weitere Vorteile und Merkmale der Erfindung ergeben sich aus der folgenden Beschreibung in Verbindung mit den Figuren der Zeichnungen.

Die Erfindung wird nachfolgend an Ausführungsbeispielen anhand der Zeichnungen näher erläutert. Dabei sind funktionsbeziehungsweise wirkungsgleiche Elemente, Bereiche und Strukturen mit den gleichen Bezugszeichen versehen. Insoweit sich Elemente, Bereiche oder Strukturen in ihrer Funktion entsprechen, wird deren Beschreibung nicht zu jedem der Ausführungsbeispiele wiederholt.

In den Zeichnungen zeigen:
- Figur 1: eine schematische Darstellung eines Etiketts gemäß einer Ausführungsform der Erfindung in einer per- spektivischen Seitensicht,
- Figur 2: eine schematische Darstellung eines Etiketts gemäß einer weiteren Ausführungsform der Erfindung in ei- ner Draufsicht,
- Figur 3A: eine schematische Darstellung eines Etiketts gemäß einer weiteren Ausführungsform der Erfindung in ei- ner Draufsicht,
- Figur 3B: eine schematische Darstellung eines Etiketts gemäß einer weiteren Ausführungsform der Erfindung in ei- ner Draufsicht,
- Figur 3C: eine schematische Darstellung eines Etiketts gemäß einer weiteren Ausführungsform der Erfindung in ei- ner Draufsicht,
- Figur 4: eine schematische Darstellung eines Behälters gemäß einer Ausführungsform der Erfindung in einer Quer- schnittsansicht,
- Figur 5: eine schematische Darstellung eines Behälters mit einem Etikett gemäß einer Ausführungsform der Er- findung in einer Querschnittsansicht,
- Figur 6: eine weitere schematische Darstellung eines Behäl- ters mit einem Etikett gemäß einer Ausführungsform der Erfindung in einer Querschnittsansicht, und
- Figur 7: eine schematische Darstellung einer medizinischen Injektionsvorrichtung gemäß einer Ausführungsform der Erfindung in einer Querschnittsansicht.

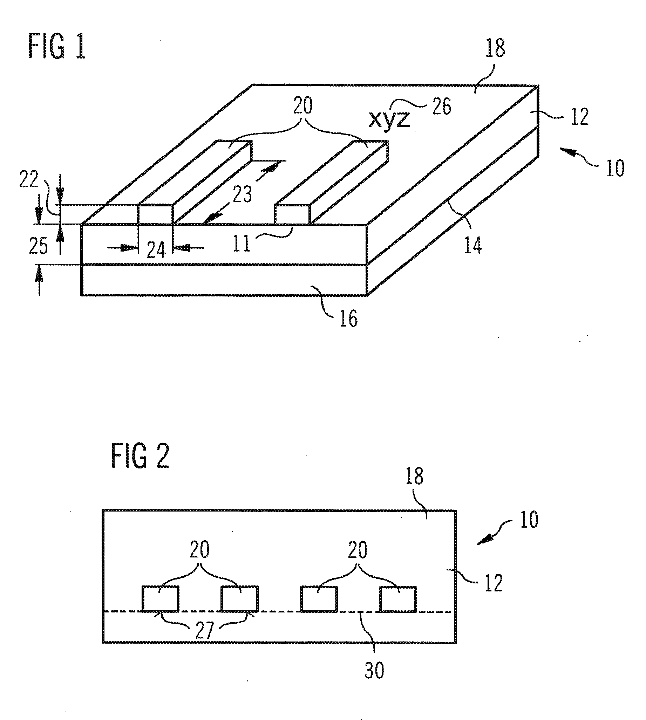

Unter Bezugnahme auf Figur 1 wird im Folgenden eine erste Ausführungsform eines erfindungsgemäßen Etiketts 10 in einer perspektivischen Seitenansicht erläutert. Das Etikett 10 umfasst eine flexible Trägerschicht 12. Die Trägerschicht 12 kann aus einer Vielzahl von Materialien hergestellt sein. So ist es beispielsweise denkbar, die Trägerschicht 12 als Papier- oder Kunststoffschicht auszuführen. Im Rahmen der Erfindung ist es ebenso möglich, die Trägerschicht 12 in einem mehrschichtigen Aufbau, beispielsweise als Laminat, herzustellen.

Auf einer Seite 14, beispielsweise einer Unterseite, der flexiblen Trägerschicht 12 ist eine Klebeschicht 16 angeordnet. Die Klebeschicht 16 kann die Seite 14 der flexiblen Trägerschicht 12 teilweise oder vollständig bedecken. Auf einer Seite 18, beispielsweise einer Oberseite, der flexiblen Trägerschicht 12 sind mehrere erhabene Strukturelemente 20 angeordnet. Erhaben bedeutet in diesem Zusammenhang, dass die Strukturelemente 20 eine Höhe 22 aufweisen, die die Dicke 25 der Trägerschicht 12 deutlich übersteigt. Bei den erhabenen Strukturelementen 20 handelt es sich also nicht nur um eine lediglich geringfügig aufgeraute Oberfläche der Trägerschicht 12, wie sie beispielsweise beim Aufbringen einer Druckfarbe auf das Etikett 10 oder wie sie beispielsweise beim Aufbringen von Symbolen einer Blindenschrift auf das Etikett entstünde.

Im Falle des Aufbringens des Etiketts 10 auf ein Behältnis, beispielsweise eine zylindrische Medikamentenampulle, sind die erhabenen Strukturelemente 20 vom Behältnis weggerichtet, wenn das Etikett 10 mittels der Klebeschicht 16 auf dem Behältnis befestigt wird. Die Strukturelemente 20 können beispielsweise Anschlagelemente bilden, die in weitere Anschlagelemente eingreifen oder in einem einfachen Fall an weiteren Anschlagelementen anliegen. Die weiteren Anschlagelemente können zum Beispiel an der Innenwand eines Hohlzylinders, beispielsweise eines Spritzengehäuses, angeordnet sein. Beim Einführen des Behältnisses in den Hohlzylinder wird zwischen den Strukturelementen des Etiketts und den weiteren Strukturelementen ein mechanischer Formschluss gebildet. Folglich entsteht ein Etikett 10, das mittels der erhabenen Strukturelemente 20 eine Haltefunktion zwischen dem Behältnis und dem Spritzengehäuse ausüben kann.

Die Höhe 22 des Strukturelements kann größer als das Vierfache der Trägerschicht 12 sein. Wenn die Trägerschicht beispielsweise eine Dicke von 100 µm hat, kann das Strukturelement eine Höhe von mehr als 0,4 mm aufweisen. Eine gute Haltefunktion wird erzielt, wenn die Höhe der Strukturelemente vorzugsweise zwischen dem Vierfachen und Zwanzigfachen der Trägerschicht beträgt. Die Strukturelemente können beispielsweise mit einer Höhe zwischen 0,4 mm und 2 mm von der Oberfläche der Trägerschicht empor stehen, insbesondere, wenn die Trägerschicht eine Höhe von 100 µm aufweist. Dadurch kann mit hoher Wahrscheinlichkeit gewährleistet werden, dass eine Medikamentenampulle, an der das Etikett befestigt ist, nicht aus einem Gehäuse einer Injektionsvorrichtung, beispielsweise einer Spritze, herausgedrückt wird.

In Figur 1 sind die erhabenen Strukturelemente 20 schematisch mit einem rechteckförmigen Querschnitt eingezeichnet. Es ist jedoch auch denkbar, die erhabenen Strukturelemente 20 mit einem anderen Querschnitt, beispielsweise mit einem quadratischen oder runden Querschnitt, auszubilden.

Die Strukturelemente können beispielsweise quaderförmige oder zylinderförmige Vorsprünge sein, die von der Seite 18 der Trägerschicht 12 hervor ragen. Bei der in Figur 1 gezeigten Ausführungsform sind die Strukturelemente 20 mit einer Seitenfläche, die durch die Längsseiten 23 und die Breitseiten 24 der Strukturelemente begrenzt sind, auf der Trägerschicht aufgebracht. Um einen sicheren mechanischen Formschluss zwischen den Strukturelementen des Etiketts und weiteren Strukturelementen zu ermöglichen, können die auf der Trägerschicht 12 aufgebrachten Strukturelemente des Etiketts 10 beispielsweise eine Höhe aufweisen, die mindestens doppelt so groß ist, wie die kürzere der Längs- und der Breiteseite 23, 24 des jeweiligen Strukturelements 20.

Die Vorsprünge können beispielsweise an ihrem von der Trägerschicht abstehenden Ende eine Verdickung aufweisen. Bei einer möglichen Ausführungsform können die Strukturelemente beispielsweise pilzförmig mit einer Verdickung am von der Trägerschicht weggerichteten Ende ausgeführt sein. Die Verdickung an dem von der Trägerschicht weggerichteten Ende eines Strukturelements dient zum Eingreifen in ein zu dem Strukturelement des Etiketts invers ausgebildeten weiteren Strukturelement, so dass zwischen beiden Strukturelementen eine formschlüssige Verbindung hergestellt werden kann.

Die Verbindung kann beispielsweise durch ein Einrasten eines Strukturelements in ein weiteres Strukturelement erfolgen. Des Weiteren kann das Strukturelement derart ausgebildet sein, dass das Strukturelement, das auf der Trägerschicht des Etiketts angeordnet ist, mit einem weiteren Strukturelement, das auf einem anderen Gegenstand angebracht ist, einen Bajonett-Verschluss bildet. Somit kann eine formschlüssige, stabile Verbindung zwischen dem Etikett beziehungsweise dem Körper, auf dem das Etikett angeordnet ist, und dem anderen Gegenstand hergestellt werden.

Die erhabenen Strukturelemente 20 können an den Seitenflächen abgeschrägt oder abgerundet sein. Die Strukturelemente weisen vorzugsweise scharfe Kanten auf, durch die ein Eingreifen eines Strukturelements in ein weiteres Strukturelement sichergestellt werden kann.

Um die oben erwähnte Haltefunktion ausüben zu können, können die erhabenen Strukturelemente 20 beispielsweise als Kunststoffsteg auf die Trägerschicht 12 aufgebracht sein. Dies kann beispielsweise mittels einer Verklebung erfolgen. Es ist aber auch möglich, die erhabenen Strukturelemente 20 als ein- oder mehrlagige Folienschicht auszubilden. Die Form der erhabenen Strukturelemente 20 kann beispielsweise mittels eines Stanzprozesses vorgegeben werden. Die ausgestanzten Umrisse der Folienschicht können auf die Trägerschicht 12 übertragen werden, indem sie beispielsweise mit der Trägerschicht 12 verklebt werden.

In einer weiteren Ausführungsform ist es vorgesehen, die erhabenen Strukturelemente 20 mittels einer Lackschicht aufzudrucken. Um die gewünschten erhabenen Strukturelemente 20 zu erzeugen, kann beispielsweise ein Lack verwendet werden, wie er als Druckfarbe für Blindenschrift bekannt ist. Im Unterschied zur Blindenschrift wird der Lack mit einer größeren Dicke auf die Trägeschrift aufgetragen, als dies beim Aufbringen von Symbolen in Blindenschrift der Fall ist. Die Strukturelemente können im Gegensatz zur Blindenschrift stempelförmige Vorsprünge sein, die rechtwinkelig zu der Oberfläche der Trägerschicht 12 von der Trägerschicht empor ragen.

Darüber hinaus kann das Etikett 10 beispielsweise mit Symbolen 26 versehen sein, die eine Information über den Inhalt eines Behältnisses, der Verwendungsart oder andere Herstellerhinweise repräsentieren. Diese Symbole werden üblicherweise auf die flexible Trägerschicht 12 aufgedruckt. Wie in Figur 1 zu sehen ist, repräsentieren die Buchstaben "xyz" die Symbole 26 auf dem Etikett 10.

Unter Bezugnahme auf Figur 2 wird im Folgenden eine weitere Ausführungsform eines erfindungsgemäßen Etiketts 10 in einer Draufsicht näher beschrieben. Das Etikett 10 weist eine Vielzahl von erhabenen Strukturelementen 20 auf, die wie in Figur 1 gezeigt ist, auf der Oberseite 18 der flexiblen Trägerschicht 12 angeordnet sind.

Alle der auf der Trägerschicht 12 aufgebrachten Strukturelemente 20 können die gleiche Form aufweisen. Die Strukturelemente können in der Weise auf der Trägerschicht angeordnet sein, dass jeweils eine jeweilige Seitenfläche 27 der Strukturelemente in einer Ebene liegt. Diese Seitenflächen 27 können Anschlagflächen der Strukturelemente 20 bilden, an denen weitere Strukturelemente formschlüssig anliegen. Gemäß der Ausführungsform in Figur 2 sind die Strukturelemente 20 beispielsweise parallel zueinander angeordnet. Die parallelen Strukturelemente 20 weisen dabei jeweils einen Startpunkt auf, der entlang einer Linie 30 angeordnet ist. Folglich kann ein mit dem Etikett 10 versehenes Behältnis die erhabenen Strukturelemente 20 als Widerlager nutzen, wobei die Anstoßfläche des Widerlagers entlang der Linie 30 definiert ist.

Des Weiteren ist in Figur 2 gezeigt, dass das Etikett 10 einen im Wesentlichen rechteckförmigen Grundriss aufweist. Es ist selbstverständlich ebenso möglich, andere Formen für das Etikett 10 zu verwenden.

Um das Etikett 10 auf einem Behältnis aufbringen zu können, ist es jedoch vorteilhaft, dass das Etikett 10 entlang der Linie 30 eine Außenabmessung aufweist, die beispielsweise einem Außenumfang des zu etikettierenden Behältnisses entspricht. Somit kann erreicht werden, dass sich im Falle des Aufbringens des Etiketts 10 keine Strukturelemente 20 überlappen.

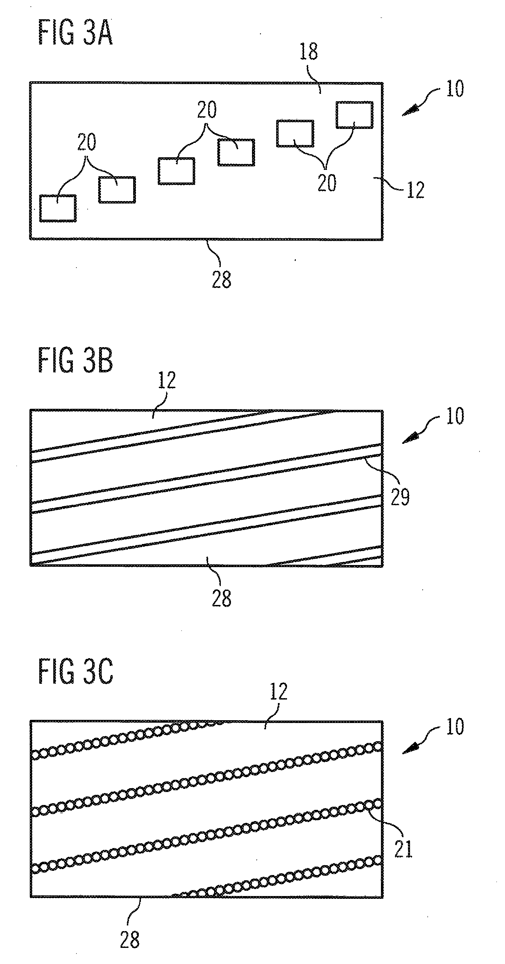

Unter Bezugnahme auf Figur 3A ist im Folgenden eine weitere Ausführungsform des Etiketts 10 gezeigt. Die in Figur 3A gezeigte Ausführungsform unterscheidet sich von der in Figur 2 beschriebenen dadurch, dass eine Vielzahl von erhabenen Strukturelementen 20 so angeordnet wird, dass sie sich im Falle des Aufbringens auf ein zylindrisches Behältnis zu einem Gewinde auf der Außenseite des Behältnisses ergänzen.

Das in Figur 3A gezeigte Ausführungsbeispiel stellt folglich eine einzelne Windung eines Gewindes dar. Es ist jedoch ebenso denkbar, mehrere Strukturelemente 20 so anzuordnen, dass sich mehrere Gewindegänge bilden lassen. Bei der in Figur 3A gezeigten Ausführungsform sind die Strukturelemente als einzelne Vorsprünge ausgebildet, die in geringem Abstand versetzt zueinander angeordnet sind. Der Abstand ist so zu wählen, dass die Vorsprünge einen Gewindegang bilden.

Bei der in Figur 3B gezeigten Ausführungsform sind auf der Oberseite 18 der Trägerschicht 12 stegförmige Strukturelemente 29 aufgebracht. Einzelne Gewindegänge des Gewindes entstehen, indem mehrere der stegförmigen Strukturelemente 29 mit einem geringen Abstand versetzt zueinander auf der Trägerschicht angeordnet werden. Die einzelnen stegförmigen Strukturelemente 29 sind bezüglich der Richtung der Seitenkante 28 des Etiketts schräg auf der Trägerschicht angeordnet. Beim Anbringen des Etiketts auf einem zylinderförmigen Behältnis bilden die einzelnen stegförmigen Strukturelemente das Gewinde.

Figur 3C zeigt eine Ausführungsform, bei der die Strukturelemente kugelförmig ausgebildet sind. Eine Vielzahl dieser kugelförmigen Strukturelemente 21 kann so angeordnet sein, dass die kugelförmigen Strukturelemente schräg zur Richtung einer Seitenkante 28 verlaufen. Aneinander angrenzende kugelförmiger Strukturelemente bildet einen Gewindegang. Die kugelförmigen Strukturelemente der einzelnen Gewindegänge sind mit einem geringen Abstand versetzt zueinander auf der Trägerschicht angeordnet. Wenn das Etikett auf einem zylinderförmigen Gegenstand aufgeklebt wird, wird durch die kugelförmigen Strukturelemente ein Gewinde gebildet. Die kugelförmigen Strukturelemente können als Kugeln aus einem Material aus Glas oder einem Material aus einem Kunststoff, beispielsweise aus einem Polymer, ausgebildet sein. Die Kugeln 21 weisen jeweils einen Durchmesser von weniger als 2 mm auf.

Die Strukturelemente der in den Figuren 1, 2, 3A, 3B und 3C gezeigten Ausführungsformen können beispielsweise durch eine Klebeschicht 11 auf der Trägerschicht 12 aufgebracht sein. Der Kleber kann ein Material enthalten, das bei Wärmeeinwirkung oder bei Bestrahlung, beispielsweise mittels UV-Licht, aushärtet.

In einer weiteren Ausführungsform, die nicht in Figur 2 oder Figur 3 gezeigt ist, kann beispielsweise die Oberseite des erhabenen Strukturelements 20 derartig mit einer Struktur versehen werden, dass sich die Oberseiten der Strukturelemente 20 zu einem Gewinde zusammenfügen. Dazu ist es beispielsweise denkbar, die Oberseite der Strukturelemente 20 mit einer Strukturierung zu versehen.

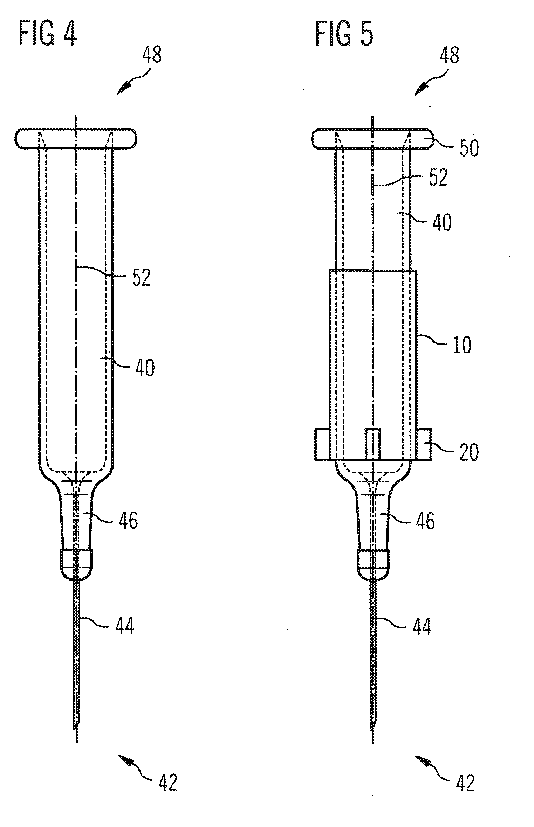

Im Folgenden wird die Verwendung der erhabenen Strukturelemente 20 auf dem Etikett 10 näher erläutert. Unter Bezugnahme auf Figur 4 ist ein zylindrisches Behältnis 40 gezeigt, auf dem das Etikett 10 angebracht werden kann. Das Behältnis 40 ist beispielsweise eine Kartusche für ein medizinisches Injektionsgerät, wie zum Beispiel ein Injektionspen oder dergleichen. Dazu weist das Behältnis 40 an seinem distalen Ende 42 eine Nadel 44 auf.

Die Nadel 44 ist über einen als Nadelhalter ausgebildeten Flansch 46 an das Behältnis 40 angefügt. Am proximalen Ende 48 des Behältnisses 40 befindet sich beispielsweise ein Fingerflansch 50. Das Behältnis 40 ist im Wesentlichen zylindrisch, wobei die Zylinderachse 52 als gestrichelte Linie in Figur 4 eingezeichnet ist. Der Fingerflansch 50 erstreckt sich üblicherweise radial, das heißt senkrecht zur Zylinderachse 52 nach außen.

Unter Bezugnahme auf Figur 5 ist das Behältnis 40 gezeigt, nachdem es mit dem Etikett 10 in einer Ausführungsform gemäß Figur 2 bestückt wurde. Die erhabenen Strukturelemente 20, die an der dem distalen Ende 42 des Behälters 40 zugewandten Seite des Etiketts 10 angeordnet sind, weisen dabei radial nach außen. Die Strukturelemente 20 sind also vom Behälter 40 weggerichtet.

Wie in Figur 5 zu sehen ist, weist somit ein Behältnis 40, das mit dem erfindungsgemäßen Etikett 10 bestückt ist, neben dem Fingerflansch und der Nadelhalterung 46 noch die Strukturelemente 20 auf, die als Widerlager für die Befestigung des Behältnisses 40 in einem Gehäuse herangezogen werden können. Es versteht sich jedoch von selbst, dass die Erfindung nicht auf die in Figur 5 gezeigte Anwendung beschränkt ist. Beispielsweise kann das Etikett 10 auch bei einem Behältnis 40 eingesetzt werden, das überhaupt keinen Fingerflansch aufweist.

Zusammenfassend ergibt sich aufgrund der Haltefunktion, die von den erhabenen Strukturelementen 20 ausgeübt werden, allein oder in Kombination mit weiteren Haltepunkten eine einfache Befestigungsmöglichkeit für ein Behältnis 40 innerhalb eines Gehäuses. Das Etikett 10 kann dabei entweder bereits vorhandene Halterungen unterstützen oder aber als einzige Halterungsmöglichkeit vorgesehen sein.

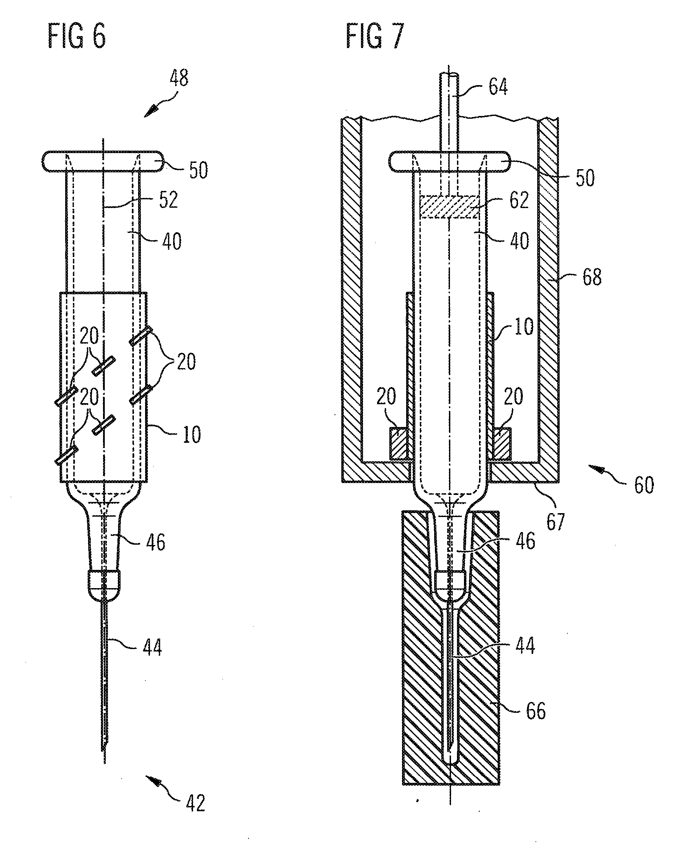

Unter Bezugnahme auf Figur 6 wird im Folgenden ein Behältnis 40 beschrieben, das mit einem Etikett 10 ähnlich zur Ausführungsform gemäß Figur 3 bestückt ist. Im Unterschied zu der in Figur 5 gezeigten Ausführungsform bilden hierbei die erhabenen Strukturelemente 20 ein Gewinde auf der Außenseite des Behältnisses 40. Folglich kann das Behältnis 40 in einem Gehäuse beispielsweise positioniert oder befestigt werden. Dazu müssten an dem Gehäuse entsprechende Perforierungen gebildet sein, die ein Innengewinde bilden.

Unter Bezugnahme auf Figur 7 wird im Folgenden eine medizinische Injektionsvorrichtung beschrieben, die von der Erfindung Gebrauch macht. Die Injektionsvorrichtung 60 ist beispielsweise ein Injektionspen, der üblicherweise zur Selbstmedikamentation eingesetzt wird. Es ist aber auch denkbar, dass die medizinische Injektionsvorrichtung 60 eine Spritze für andere Anwendungsfälle darstellt. Die Injektionsvorrichtung 60 umfasst das Behältnis 40 in Form einer Medikamentenkartusche.

Zusätzlich zu den bereits im Zusammenhang mit den Figuren 4 bis 6 erläuterten Merkmalen ist in Figur 7 ein Kolben 62 und eine Kolbenstange 64 dargestellt, die zur Verabreichung des im Behältnis 40 eingebrachten Medikaments dienen. Wie in der Technik allgemein bekannt ist, kann die Kolbenstange 64 mit einem geeigneten Mechanismus zur Dosisabgabe versehen sein. Da der Mechanismus zur Dosisabgabe nicht Gegenstand der Erfindung ist, wird im Folgenden auf nähere Erläuterungen verzichtet.

Üblicherweise werden medizinische Injektionsvorrichtungen 60, die an ihrem distalen Ende mit einer Nadel versehen sind, durch eine Nadelschutzvorrichtung 66 gegen unbeabsichtigte Berührungen geschützt. Wie aus Figur 7 zu erkennen ist, kann die Nadelschutzvorrichtung 66 dabei bis an die Schulter reichen, die zwischen den Außenwänden des Behältnisses 40 und der Nadelhalterung 46 gebildet wird. Um das Behältnis 40 nun sicher im Gehäuse 68 befestigen zu können, ist ein Widerlager in Form der erhabenen Strukturelemente 20 auf dem Etikett 10 vorgesehen. Folglich kann beim Vortrieb des Kolbens 62 über die Kolbenstange 64 das Behältnis 40 nicht aus dem Gehäuse 68 rutschen.

Wie bereits im Zusammenhang mit Figur 5 erläutert, kann dabei ein Fingerflansch die Befestigung des Behältnisses 40 am Gehäuse 68 unterstützen. Das Etikett 10 lässt sich jedoch auch bei Behältnissen anwenden, die keinen Fingerflansch aufweisen. Es wäre ebenso denkbar, dass der Fingerflansch durch ein weiteres Etikett 10 (nicht in Figur 7 gezeigt) ersetzt wird. Zusammenfassend wird gemäß der Erfindung eine Rückhaltung eines beispielsweise medizinischen Behältnisses durch Formschluss in einem Gehäuse erreicht.

Insbesondere bei Verwendung einer Nadelschutzvorrichtung kann es vorkommen, dass deren Durchmesser größer ist als der Durchmesser des Behältnisses 40. Dadurch kann die am Übergang zwischen Behältnis und Nadel liegende Schulter nicht mehr als Anschlag verwendet werden. Die alleinige Verwendung des Fingerflansches würde die Toleranzen der Herstellung eines Injektionspens erhöhen. Im schlimmsten Fall könnte es vorkommen, dass bei Auslösung des Injektionspens die gesamte Spritze aus dem Gehäuse gedrückt wird, beispielsweise aufgrund einer Materialschwäche.

Gemäß der Erfindung wird dies verhindert und eine Positionierung und/oder Fixierung mittels eines definierten Anschlags oder eines Gewindes erreicht. Da medizinische Behältnisse üblicherweise mit einem Etikett zur Kennzeichnung ihres Inhalts versehen werden, wird gemäß der Erfindung die Komplexität der Herstellung eines Injektionspens nicht erhöht.

Die Erfindung ist nicht durch die Beschreibung anhand der Ausführungsbeispiele beschränkt. Vielmehr umfasst die Erfindung jedes neue Merkmal sowie jede Kombination von Merkmalen, was insbesondere jede Kombination von Merkmalen in den Patentansprüchen beinhaltet, auch wenn dieses Merkmal oder diese Kombination selbst nicht explizit in den Patentansprüchen oder Ausführungsbeispielen angegeben ist.

### Bezugszeichenliste:

- 10: Etikett
- 12: flexible Trägerschicht
- 14: Unterseite
- 16: Klebeschicht
- 18: Oberseite
- 20: erhabene Strukturelemente
- 21: kugelförmiges Strukturelement
- 22: Höhe
- 23: Breite
- 24: Länge
- 25: Dicke
- 26: Symbole
- 27: Seitenfläche des Strukturelements
- 28: Seitenkante
- 29: stegförmiges Strukturelement
- 30: Linie der Startpunkte
- 40: Gehäuse
- 42: distales Ende
- 44: Nadel
- 46: Nadelhalterung
- 48: proximales Ende
- 50: Fingerflansch
- 52: Zylinderachse
- 60: Injektionsvorrichtung
- 62: Kolben
- 64: Kolbenstange
- 66: Nadelschutzvorrichtung
- 68: Gehäuse

## Patentansprüche

1. Etikett (10) zum Etikettieren eines Behältnisses (40) umfassend eine flexible Trägerschicht (12), die auf einer Seite (18) wenigstens teilweise mit wenigstens einem erhabenen Strukturelement (20) versehen ist, **dadurch gekennzeichnet, dass** das Strukturelement (20) derart ausgeführt ist, dass im Falle des Aufbringens des Etiketts auf das Behältnis (40) das Strukturelement (20) vom Behältnis (40) weggerichtet ist und wobei das Strukturelement (20) dazu geeignet ist, mit einem weiteren Strukturelement (67) eine formschlüssige Verbindung zu bilden.

2. Etikett nach Anspruch 1, bei dem das Strukturelement (20) eine Höhe (22) aufweist, die größer als das Vierfache der Trägerschicht (12) ist.

3. Etikett nach einem der Ansprüche 1 oder 2,
- bei dem das Strukturelement (20) mit einer Seitenfläche, die durch Seiten (23, 24) des Strukturelements begrenzt wird, auf der Trägerschicht (12) aufgebracht ist,
- wobei das Strukturelement (20) eine Höhe (22) aufweist, die mindestens doppelt so groß ist, wie die kürzere der Seiten (23, 24) der Seitenfläche.

4. Etikett nach einem der Ansprüche 1 bis 3, bei dem das Strukturelement mittels einer Klebeschicht (11) auf die Trägerschicht (12) geklebt ist.

5. Etikett nach einem der Ansprüche 1 bis 4, bei dem das Strukturelement (20) als Kunststoffsteg (29) oder als strukturierte Farbschicht oder als strukturierte Folienschicht auf die Trägerschicht aufgebracht ist.

6. Etikett nach einem der Ansprüche 1 bis 5, bei dem das Strukturelement (20) im Falle des Aufbringens des Etiketts auf das Behältnis (40) als Widerlager für die Befestigung des Behältnisses (40) in einem Gehäuse (68) geeignet ist oder ein Gewinde bildet.

7. Etikett nach einem der Ansprüche 1 bis 6, bei dem eine Vielzahl der Strukturelemente (20) aus einer Vielzahl von kugelförmigen Elementen (21) gebildet wird, wobei die kugelförmigen Elemente derart auf der Seite (18) der Trägerschicht (12) angeordnet sind, dass die kugelförmigen Elemente (21) im Falle des Aufbringens des Etiketts auf das Behältnis das Gewinde bilden.

8. Medizinische Injektionsvorrichtung, umfassend:
- ein Etikett (10) nach einem der Ansprüche 1 bis 7,
- ein zylindrisches Behältnis (40) zur Aufnahme eines medizinischen Produktes,
- ein Gehäuse (68), in dem das Behältnis (40) angeordnet ist, **dadurch gekennzeichnet, dass**
- das Behältnis (40) mit dem Etikett in der Weise versehen ist, dass das Strukturelement (20) einen vom Behältnis (40) weggerichteten Vorsprung bildet, und
- das Strukturelement (20) dazu geeignet ist, mit einem weiteren Strukturelement (67) eine formschlüssige Verbindung zu bilden.

9. Medizinische Injektionsvorrichtung nach Anspruch 8, bei der das Strukturelement (20) eine Haltefunktion in Form eines Widerlagers oder Gewindes für das zylindrische Behältnis (40) in dem Gehäuse (68) ausübt.

10. Medizinische Injektionsvorrichtung nach einem der Ansprüche 8 oder 9, bei der das Strukturelement (20) eine Höhe (22) aufweist, die größer als das das Vierfache der Trägerschicht (12) ist.

11. Medizinische Injektionsvorrichtung nach einem der Ansprüche 8 bis 10,
- bei der das Strukturelement (20) mit einer Seitenfläche, die durch Seiten (23, 24) des Strukturelements begrenzt wird, auf der Trägerschicht (12) aufgebracht ist,
- wobei das Strukturelement (20) eine Höhe (22) aufweist, die mindestens doppelt so groß ist, wie die kürzere der Seiten (23, 24) der Seitenfläche.

12. Medizinische Injektionsvorrichtung nach einem der Ansprüche 8 bis 11, bei der das Strukturelement mittels einer Klebeschicht (11) auf die Trägerschicht (12) geklebt ist.

13. Verfahren zur Herstellung eines Etiketts, das die folgenden Schritte umfasst:
- Bereitstellen einer flexiblen Trägerschicht (12), und
- Aufbringen wenigstens eines erhabenen Strukturelements (20) auf einer Seite (18) der Trägerschicht (12), **dadurch gekennzeichnet, dass** das Strukturelement (20) derart ausgeführt ist, dass im Falle des Aufbringens auf das Behältnis (40) das Strukturelement (20) vom Behältnis (40) weg gerichtet ist und wobei das Strukturelement (20) dazu geeignet ist, mit einem weiteren Strukturelement (67) eine formschlüssige Verbindung zu bilden.

14. Verfahren nach Anspruch 13, bei dem das Strukturelement (20) mit einer Höhe (22) auf der Trägerschicht (12) aufgebracht wird, die größer als das Vierfache der Trägerschicht (12) ist.

15. Verfahren nach einem der Ansprüche 13 oder 14,
- bei dem das Strukturelement (20) mit einer Seitenfläche, die durch Seiten (23, 24) des Strukturelements begrenzt wird, auf der Trägerschicht (12) aufgebracht wird,
- wobei das Strukturelement (20) mit einer Höhe (22) auf der Trägerschicht (12) aufgebracht wird, die mindestens doppelt so groß ist, wie die Kürzere der Seiten (23, 24) der Seitenfläche.

## Claims

1. A tag (10) for labeling a container (40), comprising a flexible carrier layer (12) which is provided on one side (18) at least partially with at least one raised structural element (20), **characterized in that** the structural element (20) is implemented in such a way that, in the event of applying the tag on the container (40), the structural element (20) faces away from the container (40) and is suitable to form a positive-fit connection with a further structural element (67).

2. The tag according to claim 1, in which the structural element (20) has a height (22) which is larger than the fourfold value of the carrier layer (12).

3. The tag according to any of claims 1 or 2,
- in which the structural element (20) is applied on the carrier layer (12) with a side face which is bordered by sides (23, 24) of the structural element (20),
- the structural element (20) having a height (22) which is at least twice as large as the shorter one of the sides (23, 24) of the side face.

4. The tag according to any of claims 1 to 3, in which the structural element is bonded to the carrier layer (12) by means of an adhesive layer (11).

5. The tag according to any of claims 1 to 4, in which the structural element (20) is applied on the carrier layer as a plastic web (29) or as a structured layer of paint or structured foil layer.

6. The tag according to any of claims 1 to 5, in which the structural element (20), in the event of applying the tag on the container (40), is suitable to serve as an abutment for fastening the container (40) in a housing (68) or forms a threaded portion.

7. The tag according to any of claims 1 to 6, in which a plurality of the structural elements (20) is formed from a plurality of ball-shaped elements (21), the ball-shaped elements being arranged on the side (18) of the carrier layer (12) in such a manner that the ball-shaped elements (21) form the treaded portion in the event of applying the tag on the container.

8. A medical injection device, comprising:
- a tag (10) according to any of claims 1 to 7,
- a cylindrical container (40) for receiving a medical product,
- a housing (68) in which the container (40) is disposed, **characterized in that**
- the container (40) is provided with the tag in such a way that the structural element (20) forms a protrusion facing away from the container (40), and
- the structural element (20) is suitable to form a positive-fit connection with a further structural element (67).

9. The medical injection device according to claim 8, in which the structural element (20) exerts a retaining function in the form of an abutment or a threaded portion for the cylindrical container (40) in the housing (68).

10. The medical injection device according to any of claims 8 or 9, in which the structural element (20) has a height (22) which is larger than the fourfold value of the carrier layer (12).

11. The medical injection device according to any of claims 8 to 10,
- in which the structural element (20) is applied on the carrier layer (12) with a side face which is bordered by sides (23, 24) of the structural element,
- the structural element (20) having a height (22) which is at least twice as large as the shorter one of the sides (23, 24) of the side face.

12. The medical injection device according to any of claims 8 to 11, in which the structural element is bonded to the carrier layer (12) by means of an adhesive layer (11).

13. A method of producing a tag, comprising the following steps:
- providing a flexible carrier layer (12), and
- applying at least one raised structural element (20) on one side (18) of the carrier layer (12), **characterized in that** the structural element (20) is realized such that, in the event of applying it on the container (40), the structural element (20) faces away from the container (40) and is suitable to form a positive-fit connection with a further structural element (67).

14. The method according to claim 13, in which the structural element (20) is applied on the carrier layer (12) with a height (22) which is larger than the fourfold value of the carrier layer (12).

15. The method according to any of claims 13 or 14,
- in which the structural element (20) is applied on the carrier layer (12) with a side face which is bordered by sides (23, 24) of the structural element,
- the structural element (20) being applied on the carrier layer (12) with a height (22) which is at least twice as large as the shorter one of the sides (23, 24) of the side face.

## Revendications

1. Étiquette (10) destinée à étiqueter un récipient (40) comprenant une couche support flexible (12) qui est pourvue sur un côté (18) au moins en partie d'au moins un élément de structure (20) en relief, **caractérisée en ce que** l'élément de structure (20) est réalisé de telle façon qu'en cas d'apposition de l'étiquette sur le récipient (40), l'élément de structure (20) est dirigé en éloignement du récipient (40) et sachant que l'élément de structure (20) est apte à constituer une liaison par complémentarité de forme avec un autre élément de structure (67).

2. Étiquette selon la revendication 1, dans laquelle l'élément de structure (20) présente une hauteur (22) qui est plus grande que le quadruple de la couche support (12).

3. Étiquette selon l'une des revendications 1 ou 2,
- dans laquelle l'élément de structure (20) est apposé sur la couche support (12) par une face latérale qui est limitée par des côtés (23, 24) de l'élément de structure,
- sachant que l'élément de structure (20) présente une hauteur (22) qui est au moins le double du plus court des côtés (23, 24) de la face latérale.

4. Étiquette selon l'une des revendications 1 à 3, dans laquelle l'élément de structure est collé sur la couche support (12) au moyen d'une couche adhésive (11).

5. Étiquette selon l'une des revendications 1 à 4, dans laquelle l'élément de structure (20) est apposé sur la couche support comme traverse en matière plastique (29) ou comme couche de peinture structurée ou comme couche de film structurée.

6. Étiquette selon l'une des revendications 1 à 5, dans laquelle l'élément de structure (20), en cas d'apposition de l'étiquette sur le récipient (40), est adapté comme butée pour la fixation du récipient (40) dans un boîtier (68) ou constitue un filetage.

7. Étiquette selon l'une des revendications 1 à 6, dans laquelle une pluralité des éléments structurels (20) est constituée à partir d'une pluralité d'éléments de forme sphérique (21), sachant que les éléments de forme sphérique sont disposés sur le côté (18) de la couche support (21) de telle façon qu'en cas d'apposition de l'étiquette sur le récipient, les éléments de forme sphérique constituent un filetage.

8. Dispositif médical d'injection, comprenant :
- une étiquette (10) selon l'une des revendications 1 à 7,
- un récipient cylindrique (40) destiné à accueillir un produit médical,
- un boîtier (68) dans lequel le récipient (40) est disposé, **caractérisé en ce que**
- le récipient (40) est pourvu de l'étiquette de telle manière que l'élément de structure (20) forme une saillie dirigée en éloignement du récipient (40), et
- l'élément de structure (20) est apte à constituer une liaison par complémentarité de forme avec un autre élément de structure (67).

9. Dispositif médical d'injection selon la revendication 8, dans lequel l'élément de structure (20) remplit une fonction de maintien sous forme d'une butée ou d'un filetage pour le récipient cylindrique (40) dans le boîtier (68).

10. Dispositif médical d'injection selon l'une des revendications 8 ou 9, dans lequel l'élément de structure (20) présente une hauteur (22) qui est plus grande que le quadruple de la couche support (12).

11. Dispositif médical d'injection selon l'une des revendications 8 à 10,
- dans lequel l'élément de structure (20) est apposé sur la couche support (12) par une face latérale qui est limitée par des côtés (23, 24) de l'élément de structure,
- sachant que l'élément de structure (20) présente une hauteur (22) qui est au moins le double du plus court des côtés (23, 24) de la face latérale.

12. Dispositif médical d'injection selon l'une des revendications 8 à 11, dans lequel l'élément de structure est collé sur la couche support (12) au moyen d'une couche adhésive (11).

13. Procédé de fabrication d'une étiquette, lequel comprend les étapes suivantes :
- mise à disposition d'une couche support (12) flexible, et
- apposition d'au moins un élément de structure (20) en relief sur un côté (18) de la couche support (12), **caractérisé en ce que** l'élément de structure (20) est réalisé de telle façon qu'en cas d'apposition sur le récipient (40), l'élément de structure (20) est dirigé en éloignement du récipient (40) et sachant que l'élément de structure (20) est apte à constituer une liaison par complémentarité de forme avec un autre élément de structure (67).

14. Procédé selon la revendication 13, dans lequel l'élément de structure (20) est apposé sur la couche support (12) avec une hauteur (22) qui est plus grande que le quadruple de la couche support (12).

15. Procédé selon l'une des revendications 13 ou 14,
- dans lequel l'élément de structure (20) est apposé sur la couche support (12) par une face latérale qui est limitée par des côtés (23, 24) de l'élément de structure,
- sachant que l'élément de structure (20) est apposé sur la couche support (12) avec une hauteur (22) qui est au moins le double du plus court des côtés (23, 24) de la face latérale.
